Europäisches Patentamt

European Patent Office

Office européen des brevets

(19)

(11) Veröffentlichungsnummer: **0 177 629**
**B1**

(12)

# EUROPÄISCHE PATENTSCHRIFT

(45) Veröffentlichungstag der Patentschrift:
**14.09.88**

(51) Int. Cl.⁴: **G 01 N 27/74**

(21) Anmeldenummer: **84112178.3**

(22) Anmeldetag: **11.10.84**

(54) Verfahren und Vorrichtung zum Messen des Konzentrationsunterschiedes an paramagnetischen Bestandteilen von Gasen.

(43) Veröffentlichungstag der Anmeldung:
**16.04.86 Patentblatt 86/16**

(45) Bekanntmachung des Hinweises auf die Patenterteilung:
**14.09.88 Patentblatt 88/37**

(84) Benannte Vertragsstaaten:
**AT BE CH DE FR GB IT LI NL SE**

(56) Entgegenhaltungen:
**DE - A - 2 702 516**
**DE - A - 3 313 537**
**GB - A - 1 236 825**
**GB - A - 1 427 515**
**US - A - 3 049 665**

(73) Patentinhaber: **LEYBOLD AKTIENGESELLSCHAFT,**
**Wilhelm-Rohn-Strasse 25, D-6450 Hanau 1 (DE)**

(72) Erfinder: **Hummel, Heinz, Dr., Fasanenweg 14,**
**D-6240 Königstein-Johanniswald (DE)**

(74) Vertreter: **Zapfe, Hans, Dipl.-Ing.,**
**Seestrasse 2 Postfach 30 04 08, D-6054 Rodgau-3 (DE)**

**Beschreibung**

Die Erfindung betrifft ein Verfahren zum Messen des Konzentrationsunterschiedes an paramagnetischen Bestandteilen zwischen einem Messgas und einem Vergleichsgas durch periodisch abwechselndes Einbringen beider Gase in den Luftspalt eines Magnetkreises und durch Erfassung der durch die Gase verursachten periodischen Änderungen des magnetischen Flusses im Magnetkreis.

Bei den genannten Gasen handelt es sich je nach den Gegebenheiten entweder um reine Gase oder auch um Gasgemische. Ein derartiges Verfahren ist durch die US-A-3 049 665 bekannt. Durch periodische Wechselfüllung eines magnetischen Spaltraumes mit einem Vergleichsgas bekannter Zusammensetzung und einem Messgas unbekannter Zusammensetzung wird ein periodischer magnetischer Wechselfluss erzeugt, der als Mass für den Konzentrationsunterschied an paramagnetischen Bestandteilen im Messgas verwendet wird. Die bekannte Methode besitzt wesentliche Nachteile. Zur Erzeugung der Wechselfüllung werden gesteuerte Ventile oder Gasumschalter verwendet. Dies ist nachteilig hinsichtlich des dafür erforderlichen Aufwandes und der Störanfälligkeit solcher Elemente. So beträgt bei einer technisch zweckmässigen Modulationsfrequenz von 25 Hertz die Zahl der Umschaltungen in einem Betriebsjahr ca. $800 \cdot 10^6$. Ausserdem müsste bei den genannten Anordnungen der Messspaltraum einschliesslich der Zuleitungen durch grosse Volumina von neuem Gas (Messbzw. Vergleichsgas) bei jeder Wechselfüllung freigespült werden. Bei einem angenommenen Volumen von 1 cm³ für Spaltraum und Zuleitung und bei der oben angegebenen Frequenz von 25 Hertz, ergäbe sich ein Verbrauch von 90 Litern pro Stunde. Dies ist einerseits ungünstig wegen der notwendigen Bereitstellung grosser Mengen an Vergleichsgas (pro Jahr ca. 800 m³) und wegen des mit dem Messgasdurchsatz notwendigerweise verknüpften Einbringens von Schmutzanteilen in die Umschaltventile und in den Messspaltraum, was dann zu sehr hohen Anforderungen an die Probenaufbereitung führt. Durch diese Probenaufbereitung ergibt sich neben dem hierfür erforderlichen Investionsaufwand und Wartungsaufwand eine zusätzliche Zeitverzögerung für die Messung. Dies bedeutet einen weiteren Nachteil. Durch die Ventile bzw. Gasumschalter wird der Gasstrom periodisch unterbrochen, und es muss das gesamte, im Leitungssystem befindliche Gas schubweise aber restlos ausgespült werden.

Durch die DE-A-27 02 516 sind ein Verfahren und eine Vorrichtung bekannt, bei denen zur Messung der Konzentration einer Fluidkomponente aus einer Fluidsubstanz (=Probe) zwei Fluidmassen abgeleitet werden, wobei bei der einen Fluidmasse gegebenenfalls zuvor die betreffende Komponente entweder entfernt oder umgewandelt wird. Die Fluidsubstanz kann auch ein Gasgemisch sein, und an die Stelle der modifizierten Gasprobe kann auch ein von aussen zugeführtes Vergleichsgas treten. Die beiden unterschiedlichen Fluidmassen werden abwechselnd entweder mittels einer intermittierend angetriebenen Balgpumpe oder mittels einer kontinuierlich laufenden Pumpe in Verbindung mit mechanisch gesteuerten Ventilen durch ein Probenröhrchen mit einem elektrochemischen Detektor gefördert, wobei die gezogene Probe zunächst vollständig durch das Probenröhrchen hindurchgefördert wird. Bei Verwendung der Balgpumpe muss die gezogene Probe durch das Probenröhrchen hindurch bis in einen Filter oder Reaktor gesaugt werden, in dem die eine Komponente zur Erzeugung eines Vergleichsgases entfernt oder umgewandelt wird. Im Anschluss daran wird die Strömungsrichtung mittels der Balgpumpe umgekehrt und die gefilterte bzw. umgewandelte Probe aus dem Probenröhrchen bis in die Atmosphäre ausgestossen. Es handelt sich um einen notwendigerweise langsam ablaufenden Vorgang mit grossen Gasverbrauch, bei dem sich eine etwaige Grenzzone zwischen den Fluidmassen durch Diffusion verwischt bzw. gar nicht erst aufbaut. Auch muss verhindert werden, dass die Balgpumpe gerade erst an die Atmosphäre ausgestossenes Gas, das sich noch im Bereich der Mündung des Röhrchen befindet, wieder ansaugt, was bei periodischen Messungen zu laufenden Fehlmessungen führen würde. Auch die Vorgänge im Filter bzw. Reaktor benötigen eine endliche Zeit.

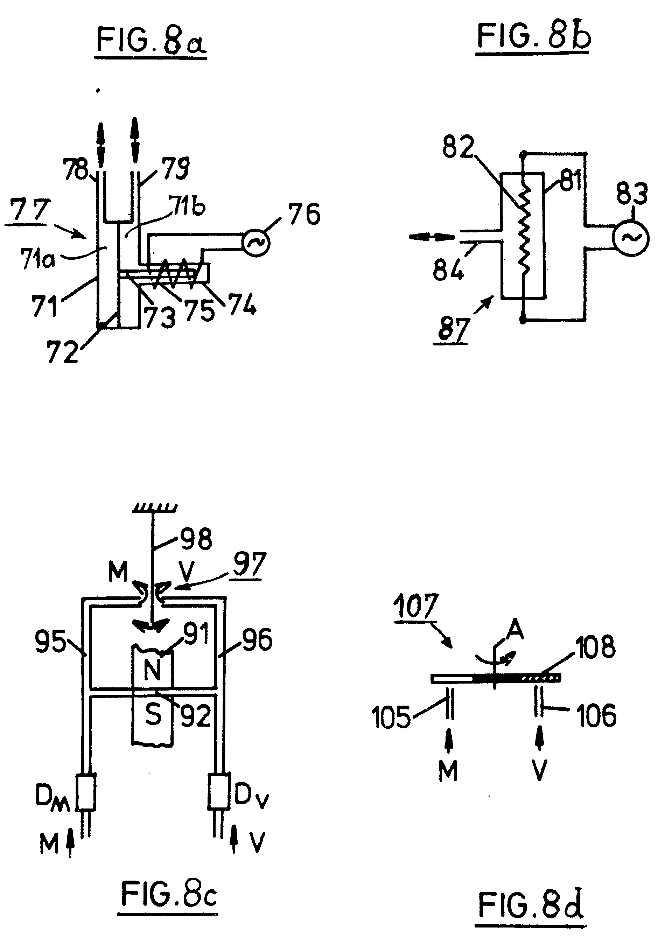

Infolgedessen ist angegeben, dass die Zykluszeit lang sein muss und etwa 1 bis 2 Minuten beträgt. Hinsichtlich des Gasaustauschs durch vollständigen Wechsel der Gasfüllung des Probenröhrchens bzw. des Wandlers ist diese Methode nicht sehr verschieden von derjenigen, die in der vorstehend genannten US-A-3 049 665 beschrieben ist. Dies gilt insbesondere auch für Vorrichtungen nach den Figuren 8 und 9 der DE-A-27 02 516, die durch eine kontinuierliche Gaszufuhr versorgt werden und bei denen der Gaswechsel mit vollständiger Spülung des Probenröhrchens bzw. Wandlers durch mechanische Umschaltventile erfolgt.

Durch die US-A-3 049 665 ist es weiterhin bekannt, das Messgas einer sogenannten Druckmodulation zu unterwerfen und die Auswirkungen periodischer Druckänderungen auf den magnetischen Fluss im Spaltraum zu messen. Dieses Verfahren weist jedoch noch weitere Nachteile auf:

1. Wollte man mit dem Wechseldruck den gleichen Messeffekt erzielen wie bei der Wechselfüllung, so müsste man mit einem Überdruck von mindestens etwa einem bar arbeiten. Bei diesem Druck sind die durch mechanische Verformung des Spaltes bedingten magnetischen Flussänderungen sehr störend und nicht ohne sehr aufwendige Zellenkonstruktionen zu vermeiden; dabei ist zu beachten, dass bei einer üblicherweise von solchen Geräten geforderten Nachweisempfindlichkeit von ca. 1‰ Sauerstoff sich nur eine magnetische Flussänderung von $2.10^{-9}$ ergibt. Spaltänderungen durch Deformation bewirken jedoch ein Vielfaches dieses Wertes an Flussänderung.

2

2. Die Mittel zur Erzeugung einer konstanten Wechseldruckamplitude sind sehr aufwendig. Ferner wird die Druckamplitude von der Messgaszusammensetzung beeinflusst.

3. Die Beschickung des System mit Messgas erfordert einen erhöhten Messgasdruck.

Durch die DE-PS 1 149 187 und die DE-PS 1 648 924 ist auch eine Art Umkehrung des Messprinzips bekannt, bei der durch periodische Änderungen der magnetischen Feldstärke proportionale Druckschwankungen in den Gasen hervorgerufen werden, die ein Mass für den Anteil an paramagnetischen Bestandteilen sind. Dabei stellt die erforderliche Wechselfeldmagnetisierung einen zusätzlichen Aufwand dar, abgesehen von den hohen Wärmeverlusten, die sich bei hinreichend grossen Wechselflussstärken ergeben.

Der Erfindung liegt die Aufgabe zugrunde, den Verbrauch insbesondere an Vergleichsgas und den Durchsatz an ggf. verunreinigtem Messgas zu verringern, sowie die Ansprechgeschwindigkeit und die Messgenauigkeit bei verringertem apparativem Aufwand zu erhöhen.

Die Lösung der gestellten Aufgabe erfolgt bei dem eingangs beschriebenen Verfahren erfindungsgemäss dadurch, dass man:

a) die beiden Gase periodisch abwechseln aus entgegengesetzten Richtungen in den Luftspalt einbringt,

b) eine Grenzzone zwischen den beiden Gasen senkrecht zu den Magnetfeldlinien periodisch im Luftspalt hin und her bewegt und

c) ständig einen Teil des Gasgemisches aus dem Bereich des Luftspalts durch Nachströmen mindestens eines der beiden Gase entweichen lässt.

Von dem Verfahren, bei denen der Wechseldruck in einem magnetischen Wechselfeld gemessen wird, unterscheidet sich die erfindungsgemässe Anordnung schon dadurch sehr vorteilhaft, dass die sehr empfindlichen und aufwendigen Wechseldruckempfänger vermieden werden. Ein grosser Vorteil der erfindungsgemässen Methode ist die äusserst geringe Zeitverzögerung für die Messung. Folgende Vorteile des Erfindungsgegenstandes sind besonders hervorzuheben:

1. Die Mittel zur Erzeugung der Wechselfüllung sind vergleichsweise sehr einfach und auch für einen Dauerbetrieb sehr betriebssicher.

2. Wie bei den weiter unten gezeigten Anordnungen zu erkennen ist, ist der Messgas- und Vergleichsgasverbrauch sehr gering, weil praktisch in dem von Strömungsdrosseln dynamisch abgeschlossenen System nur das eingeschlossene Gasvolumen hin und her schwingt (pulsiert). Der ständige Zustrom neuen Gases dient lediglich dazu, die durch Diffusion sich ansonsten verwischende Grenzzone zwischen Mess- und Vergleichsgas aufrecht zu erhalten und, was das Messgas anbetrifft, die Spaltfüllung bei zeitlich sich ändernder Gaskonzentration dem jeweiligen Messgaszustand anzupassen.

3. Wegen des Punktes 2 haben auch Änderungen in der zuströmenden Messgas- und Vergleichsgasdurchflussmenge keinen Einfluss auf den zeitlichen Verlauf der Wechselfüllung.

4. Bei den bekannten Wechselfüllungsmethoden tritt wegen der erforderlichen grossen Strömungsmenge im Messspalt wegen des Strömungswiderstandes ein die Messung störender Wechseldruck auf. Dieser wird bei der erfindungsgemässen Anordnung praktisch vermieden.

Die Erfindung betrifft ausserdem eine Vorrichtung zur Durchführung des Verfahrens nach Anspruch 1 mit einem innerhalb des Luftspalts des Magnetkreises gebildeten Spaltraum, der über mindestens eine Gasleitung mit einem Wechselströmungsgeber zum abwechselnden Einbringen des Mess- und des Vergleichsgases verbunden ist und mindestens eine Ableitung aufweist, sowie mit mindestens einer Wicklung für die Erfassung der periodischen Änderungen des magnetischen Flusses im Magnetkreis. Zur Lösung im wesentlichen der gleichen Aufgabe ist diese Vorrichtung erfindungsgemäss dadurch gekennzeichnet, dass

a) der Spaltraum auf zwei gegenüberliegenden Seiten mit je einer Gasleitung versehen ist, von denen die eine die Messgaszuleitung und die andere die Vergleichsgaszuleitung ist,

b) durch den Wechselströmungsgeber ein solches Gasvolumen bewegbar ist, dass der Spaltraum periodisch abwechselnd im wesentlichen mit dem Messgas oder mit dem Vergleichsgas füllbar ist, und

c) die mindestens eine Ableitung über den Spaltraum ständig mit mindestens einer der beiden Gasleitungen in Verbindung steht.

Bei Beachtung dieser Konstruktionsvorschrift ergibt sich, dass der gasbeschickte Teil der Messanordnung sehr einfach und robust hinsichtlich der Fertigung als auch hinsichtlich der Justierung und Handhabung aufgebaut werden kann. Die an die Messelektronik zu stellenden Anforderungen entsprechen etwa denen bei der Wechseldruckmethode (z.B. DE-PS 1149187 und DE-PS 1648924). Die Energieversorgungselektronik ist vergleichsweise sehr einfach, weil die für den Wechselströmungsgeber erforderliche elektrische Leistung um ein Vielfaches geringer ist, wie bei der Versorgung eines Magnetkreises hoher Wechselflussdichte mit elektrischer Leistung. Die für die Gleichfelderregung erforderliche Leistung ist ebenfalls vergleichsweise sehr gering, ganz abgesehen von der Möglichkeit der Permanentmagnet-Erregung.

Es ist dabei besonders vorteilhaft, wenn der Wechselströmungsgeber zwischen den Gasleitungen angeordnet ist und zwei durch eine Membran getrennte Kammern aufweist, von denen die eine mit der Gasleitung für das Messgas und die andere mit der Gasleitung für das Vergleichsgas verbunden ist, derart, dass durch eine periodisch oszillierende Membranbewegung Mess- und Vergleichsgas im Wechselrhythmus in den Spaltraum einführbar und wieder aus diesem entfernbar sind. Als Wechselströmungsgeber können aber auch verschiedene andere als technische Bauelemente verfügbare Anordnungen Verwendung finden, z.B. elektromagnetische Telefone (bzw.

Lautsprecher), elektrodynamische Telefone, elektrostatische Mikrophone, magnetostriktive Schallgeber, Thermophone, elektromagnetische Membranpumpen (nach Entfernung der Ventile) und andere entsprechende zum Stand der Technik gehörende Bauelemente wie mechanisch angetriebene Membran- oder Kolbengeber. Als Wechselfrequenzen für den Pulsationseffekt haben sich Frequenzen zwischen 2 und 100 Hz, vorzugsweise zwischen 5 und 50 Hz als brauchbar erwiesen.

Es ist zur Kleinhaltung des Gasdurchsatzes sowie zur Erzeugung definierter Gasströmungen weiterhin von Vorteil, wenn sowohl in den Zuleitungen zu den Gasleitungen als auch in der mindestens einen Ableitung je eine Strömungsdrossel angeordnet ist. Wenn hierbei dafür gesorgt ist, dass die Summe aller Strömungsquerschnitte stromabwärts stets grösser ist als die Summe aller Strömungsquerschnitte stromaufwärts, werden unerwünschte Druckschwankungen durch Änderung der Gasmengen wirksam verhindert. Die Wirkung von Strömungsdrosseln kann auch durch Gasleitungen mit geringen Strömungsquerschnitten erzielt werden. In jedem Falle ist jedoch dafür Sorge zu tragen, dass die Strömungsquerschnitte in dem Abschnitt der Vorrichtung, in dem die Gase hin und her bzw. in einem nur durch die Membran getrennten Kreis oszillieren, vergleichsweise grösser sind, so dass keine nennenswerten Strömungswiderstände vorhanden sind.

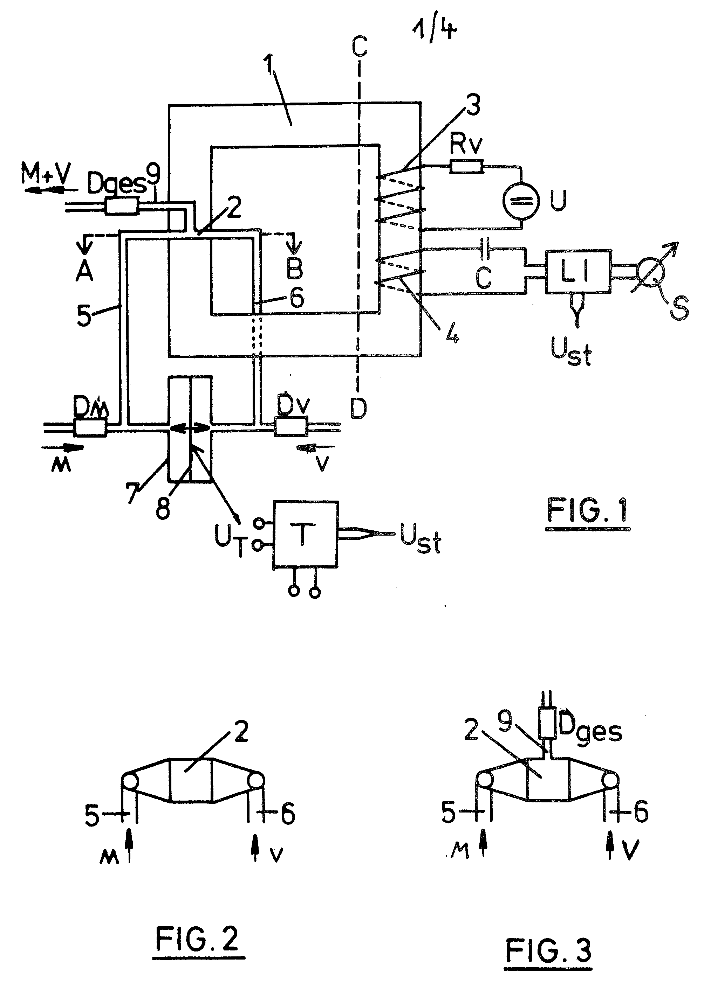

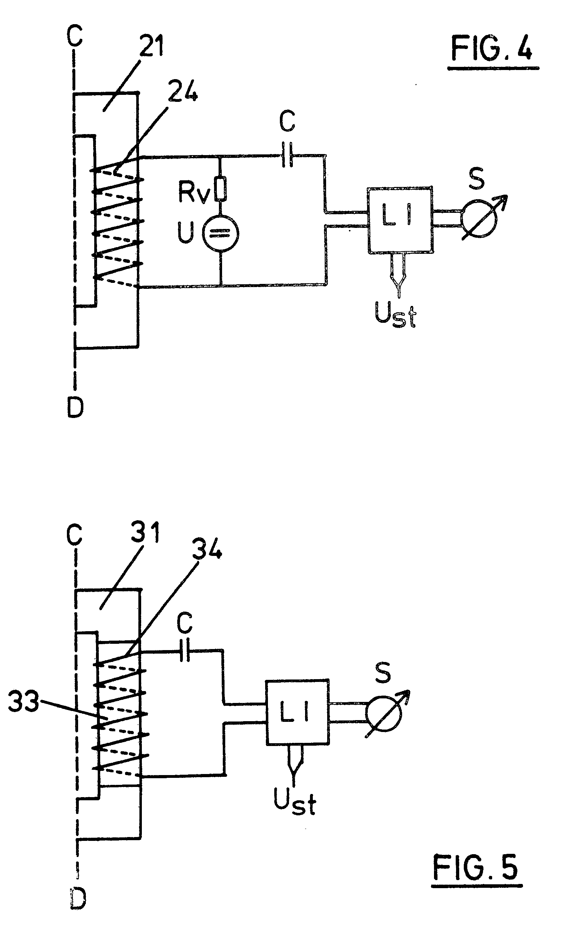

In den Figuren 1 bis 3 und 6 bis 7 sind beispielhafte Vorrichtungen gemäss der Erfindung schematisch dargestellt. Die Fig. 4 und 5 geben andere Möglichkeiten der elektrischen Beschaltung wieder. Die Fig. 8a bis 8b zeigen beispielhafte Ausführungsformen von Wechselströmungsgebern, und die Fig. 8c und 8d zeigen 2 weitere Ausführungsmöglichkeiten der Vorrichtung für die Wechselfüllung.

Die in der eingangs genannten US-A-3049665 beschriebenen Varianten: Verwendung eines Hochfrequenzfeldes, Verwendung eines Permanentmagneten, Ausführung als Topfmagnetanordnung gelten sinngemäss auch für die erfindungsgemässe Anordnung. So ist es möglich, den magnetischen Kreis aus einem Weicheisenkern mit einer Erregerwicklung aufzubauen oder innerhalb des Weicheisenkerns einen Permanentmagneten vorzusehen. Ein Hinweis darauf in allen Details erübrigt sich deshalb.

Die Fig. 1 zeigt eine Anordnung mit einem Doppelwechselströmungsgeber (zweiseitig wirkender Wechselströmungsgeber) und einem gleichstromerregten magnetischen Kreis 1. Dieser Kreis besteht aus einem aus 2 Teilen zusammengesetzten Magnetkern mit einem Spaltraum 2. Der Magnetkern ist aus weichmagnetischem Bandmaterial hergestellt und besitzt einen quadratischen Querschnitt von $1 cm^2$. Die Spaltweite beträgt 0,3 mm. Erregt wird der Magnet durch die Wicklung 3, welche über die Gleichspannungsquelle U und einen Vorwiderstand $R_V$ mit Gleichstrom beschickt wird. Der Widerstand $R_V$ soll verhindern, dass die in der Wicklung erzeugte Wechselspannung bei Wechselfüllungsmodulation kurzgeschlossen wird. Anstelle des Vorwiderstandes kann auch ein induktiver Widerstand verwendet werden. Der Spaltraum 2 ist über die Gasleitungen 5 und 6 mit dem Wechselströmungsgeber 7 mit der schwingungsfähigen Membran 8 verbunden. Die Zufuhr von Messgas (M) und Vergleichsgas (V) erfolgt über die beiden Strömungsdrosseln $D_M$ und $D_V$. Über eine zentrale Bohrung wird Mess- und Vergleichsgas aus dem Spaltraum 2 über die Ableitung 9 und die Strömungsdrossel Dges abgeleitet.

Die Figuren 2 und 3 – Schnitte entlang der Linie A-B in Fig. 1 – sollen darlegen, wie die röhrenförmigen Gaszuleitungen (Innendurchmesser ca. 2 mm) ungefähr querschnittsgleich über sich in einer Ebene kontinuierlich erweiternde und in der dazu senkrechten Ebene kontinuierlich verjüngende Übergangsstücke mit dem Spaltraum 2 verbunden sind. Die Figur 3 zeigt eine andere Ausführung für die gemeinsame Ableitung aus dem Spaltraum.

Der Spaltraum 2 wird von einer Kammer gebildet, die mit Ausnahme der Gaszu- und -ableitungen allseitig geschlossen und von planparallelen Wänden begrenzt ist, die von den magnetischen Feldlinien senkrecht durchsetzt werden. Diese Kammer ist mit enger Passung in den Luftspalt des magnetischen Kreises engesetzt.

Die Membran 8 wird über den netzgespeisten Tongenerator T mit einer Frequenz von 25 Hertz zu periodischen Schwingungen angeregt, deren Amplitude so bemessen ist, dass der resultierende Volumenhub zum Umfüllen des Spaltraumes (Volumen 30 $m^3$) mit Mess- bzw. Vergleichsgas ausreicht. Der Pfeil von der Tongeneratorspannung $U_T$ zur Membran soll die Einwirkung andeuten (siehe hierzu Erläuterung zu Fig. 8a). Die zu $U_T$ frequenzgleiche Steuerspannung $U_{St}$ (in fester Phasenbeziehung zu $U_T$) dient zur Steuerung des Lock-in-Verstärkers LI.

Eine zweite auf dem magnetischen Kern befindliche Wicklung 4 dient zur Messung des durch die Wechselfüllungsmodulation erzeugten magnetischen Wechselflusses. Die durch diesen Fluss induzierte Wechselspannung wird über den Kondensator C dem LI-Verstärker zugeleitet und nach Verstärkung und Gleichrichtung im Instrument S gemessen. Die Anzeige des Instrumentes ist dann ein Mass für den Konzentrationsunterschied an paramagnetischer Substanz (im allgemeinen Sauerstoff) zwischen Mess- und Vergleichsgas. Bei Vergleichsgas bekannter Zusammensetzung ergibt sich daraus der Gehalt an paramagnetischem Gas im Messgas.

Fig. 4 stellt im elektrischen Bereich eine Variante der Fig. 1 dar. Der nicht gezeichnete Teil – links der Linie C-D – entspricht voll der Fig. 1. Auf den Magnetkern 21 wird hier nur eine Wicklung 24, die gleichzeitig zur Felderregung (über die Gleichspannungsquelle U und den Vorwiderstand $R_V$) und zur Wechselspannungsmessung (Ankopplung an den Lock-in-Verstärker LI über den Kondensator C verwendet wird.

Die Fig. 5 ergibt sich aus Fig. 4. Die Gleichstromerregung ist fortgefallen. Ein Permanentmagnet 33 im Magnetkreis (Magnetkreis 31) erzeugt den magnetischen Gleichfluss. Die in der Wicklung 34 induzierte Wechselspannung wird wie bei Fig. 4 gemessen.

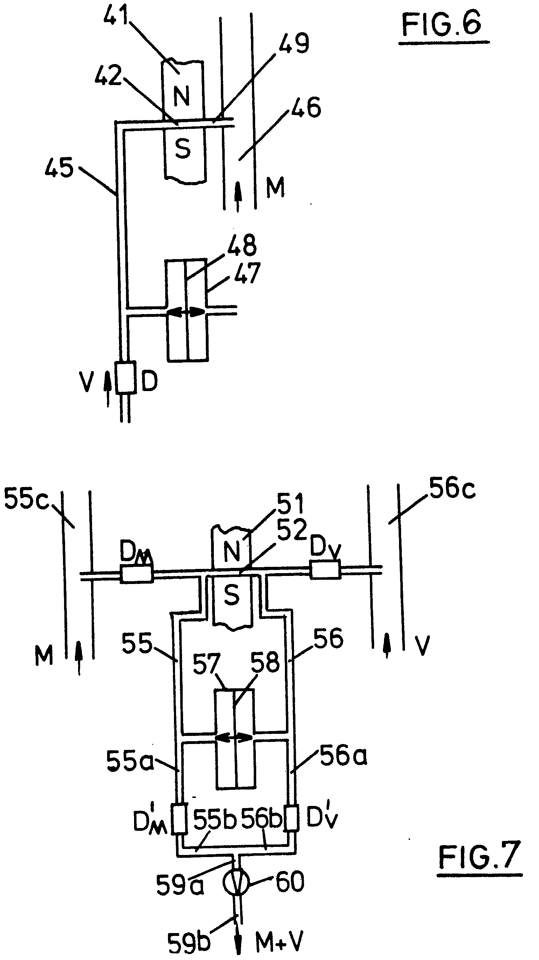

In Fig. 6 ist eine Anordnung wiedergegeben, die einen nur einseitig wirkenden Wechselströmungsgeber 47 mit einer Membran 48 verwendet. Die nicht mit dem System verbundene Seite ist zur Atmosphäre hin entlüftet. Das Vergleichsgas wird über die Drossel D und die Gasleitung 45 in den Spaltraum 42 geleitet und tritt aus diesem über die sehr kurze Gasleitung 49 (Rohr- oder Spaltstück mit möglichst geringem Volumen) in die mit Messgas beströmte Gasleitung 46, welche ihrerseits in der freien Atmosphäre endet. Der Spaltraum 42 befindet sich im Magnetkern 41, der analog zu den Figuren 1 bis 5 aufgebaut ist. Der mit der Gasleitung 45 verbundene Wechselströmungsgeber 47 saugt periodisch abwechselnd Messgas aus der Gasleitung 46 über die Gasleitung 49 in den Spaltraum 42 und verdrängt dieses wieder (im gleichen bzw. gegenphasigen Rhythmus) durch Vergleichsgas. Die Anordnung nach Fig. 6 zeichnet sich gegenüber der Anordnung nach den Figuren 1 bis 5 durch einen sehr einfachen Aufbau aus, dies gilt besonders für den Bereich des Spaltraums 42. Der Verbrauch an Vergleichsgas ist bei dieser Anordnung nach Fig. 6 zwar grösser als bei der Anordnung nach Fig. 1, jedoch ist er immer noch wesentlich kleiner als bei den bekannten Anordnungen.

Es gilt für diese wie für alle anderen in dieser Schrift beschriebenen Anordnungen, dass Mess- und Vergleichszufuhr vertauscht werden können, ohne dass sich die absolute Grösse des Messeffektes ändert.

In Fig. 7 ist eine Anordnung beschrieben, die aus der Anordnung nach Fig. 1 entsteht, wenn die gemeinsame Ableitung von Mess- und Vergleichsgas nicht direkt aus dem Spaltraum 52 heraus folgt. Die Ableitung von Mess- und Vergleichsgas aus dem aus Spaltraum 52, Gasleitungen 55 und 56 und aus Wechselströmungsgeber 57 mit Membran 58 gebildeten Wechselströmungssystem – das nach aussen wieder dynamisch über die Drosseln $D_M$ und $D_V$ abgeschlossen ist – erfolgt zunächst getrennt über die Leitungsabschnitte 55a und 56a (mit den Strömungsdrosseln $D_M'$ und $D_V'$) und dann gemeinsam über die Gasleitung 59a, die Ansaugpumpe 60 und die Ableitung 59b. Die Beschickung des ganzen Systems mit Mess- bzw. Vergleichsgas erfolgt durch Ansaugen aus den Mess- bzw. Vergleichsgas durchströmten Leitungen 55c bzw. 56c. Diese Leitungen münden in der freien Atmosphäre.

Fig. 8a gibt den beispielsweisen Aufbau eines Wechselströmungsgebers 77 wieder. In einem zylindrischen Gehäuse 71 (Zylinderachse in der Zeichenebene liegend) befindet sich in der Mittenebene senkrecht zur Zeichenebene die elastische, gasdichte Membran 72. Die Anregung der Membran erfolgt über ein von einem unmagnetischen Schutzrohr 74, umschlossenes ferromagnetisches Stäbchen 73 (welches auch magnetisiert sein kann), welches durch das in der Wicklung 75 erzeugte Wechselfeld (Wechselstromquelle 76) zu Schwingungen angeregt wird. Zur Weiterleitung der sich dabei ergebenden Volumenverschiebungen besitzt das Gehäuse 71 mit den beiden Kammern 71a und 71b die beiden Anschlussstutzen 78 und 79.

In Fig. 8b ist ein nach dem Thermophonprinzip arbeitender Wechselströmungsgeber 87 gezeigt. In dem zylindrischen Gehäuse 81 ist in der Zylinderachse ein dünner Draht oder eine aus dünnem Draht bestehende Wendel 82 gespannt. Aus der Wechselspannungsquelle 83 fliesst ein niederfrequenter Wechselstrom (Frequenz z.B. 5 Hertz) oder ein pulsierender Gleichstrom. Die dabei auftretenden periodischen Erwärmungen des Drahtes und der diesen umgebenden Luft erzeugen periodische Druckschwankungen, die zu pulsierenden Strömungen führen, welche über den Stutzen 84 der offenen Seite des Wechselströmungsgebers 47 in Fig. 6 zugeleitet werden.

Fig. 8c zeigt eine andere Form der Wechselfüllungsmodulation mit einem Wechselströmungsgeber 97 mit einer schwingenden Blattfeder 98 (Schwingungsebene gleich Zeichenebene, Blattfeder senkrecht zur Zeichenebene). Die Elemente $D_M$, $D_V$, 92, 95 und 96 entsprechen denen der Fig. 1, jedoch besitzt der Spaltraum 92 keinen besonderen Austrittsstutzen. Der magnetische Kreis 91 ist der gleiche wie in Fig. 1 (nur durch ein Teilstück angedeutet). Die Öffnungen der Gasleitungen 95 und 96 sind so zueinander und zur Blattfeder 98 angeordnet, dass beim Schwingen der Blattfeder einmal die Gasleitung 95 und eine Halbperiode später die Gasleitung 96 verschlossen ist. Im ersten Fall, wenn die Gasleitung 95 verschlossen ist, strömt das Messgas M durch den Spaltraum 92 im zweiten Fall das Vergleichsgas V. Die Blattfeder wird z.B. magnetomechanisch angetrieben und die dazu erforderliche Wechselspannung zur Steuerung der Verstärkung (wie oben beschrieben) verwendet.

Eine andere Methode der Wechselfüllung durch einen Wechselströmungsgeber 107 ist in Fig. 8d gezeigt. Sie verwendet zum periodischen, gegenphasigen Öffnen und Schliessen der Gasleitungen 105 und 106 (welche den Gasleitungen 95 und 96 in Fig. 8c entsprechen) eine vor den Gasleitungen angeordnete, synchron rotierende Segmentscheibe 108, eine Lochscheibe oder dergleichen mit der Antriebsachse A. Anstelle dieser Scheibe kann auch ein rotierendes Zahnrad verwendet werden, wobei die Gasleitungen 105 und 106 unmittelbar vor dem äusseren Zahnkranz um je einen halben Zahnabstand versetzt angeordnet sind.

Es ist auch möglich, die Wechselfüllungsmodulation ohne besonders bereitgestelltes Vergleichsgas zu betreiben. Hierbei wird das Vergleichsgas aus einem Teilstrom des Messgases dadurch gebildet, das man diesen Teilstrom unmittelbar vor dem Eintritt in den Spaltraum auf eine vorgegebene Temperatur von beispielsweise 100 °C aufheizt. An die Stelle eines Konzentrationsgradienten in der Grenzzone zwischen den beiden Gasen, die periodisch senkrecht zu den Magnetfeldlinien im Luftspalt hin und her bewegt wird, tritt hier ein Temperaturgradient, der eine vergleichbare Wirkung hat, da sich die parametrischen Eigenschaften der zur Diskussion stehenden Gase bzw. Gasbestandteile mit der Temperatur merklich ändern.

Hierzu ist es erforderlich, den Messgasstrom über zwei Drosseln in zwei Teilströme M und V zu teilen. Der Teilstrom M wird wie in Fig. 1 direkt dem Spaltraum zugeführt und der Teilstrom V über ein beheiztes Rohrstück. Dabei wird die Differenz der paramagnetischen Wirkung, welche temperaturabhängig ist, gemessen und dient unter Berücksichtigung der Temperaturdifferenz zwischen M und V zur Messung des Gehaltes an paramagnetischer Substanz im Messgas (siehe hierzu auch US-Patent 3584499).

Während bei den Wechselströmungsgebern 7, 47, 57 und 77 mit Membran und zwei durch die Membran getrennten Kammern das oszillierende Gasvolumen durch das Fördervolumen der Membran (oder eines Kolbens) bestimmt wird, ist es bei einem Wechselströmungsgeber 97 mit einer Blattfeder die Oszillationsfrequenz der Blattfeder in Verbindung mit den einzelnen Gasflüssen, welche die Grösse des oszillierenden Gasvolumens bestimmt. Analoges gilt auch für den den Wechselströmungsgeber 107 nach Fig. 8d, bei dem an die Stelle der Blattfeder offene oder geschlossene Segmente des Segmentscheibe 108 treten.

## Patentansprüche

1. Verfahren zum Messen des Konzentrationsunterschiedes an paramagnetischen Bestandteilen zwischen einem Messgas und einem Vergleichsgas durch periodisch abwechselndes Einbringen beider Gase in den Luftspalt eines Magnetkreises und durch Erfassung der durch die Gase verursachten periodischen Änderungen des magnetischen Flusses im Magnetkreis, dadurch gekennzeichnet, dass man

a) die beiden Gase periodisch abwechselnd aus entgegengesetzten Richtungen in den Luftspalt einbringt,

b) eine Grenzzone zwischen den beiden Gasen senkrecht zu den Magnetfeldlinien periodisch im Luftspalt hin und her bewegt und

c) ständig einen Teil des Gasgemischs aus dem Bereich des Luftspalts durch Nachströmen mindestens eines der beiden Gase entweichen lässt.

2. Vorrichtung zur Durchführung des Verfahrens nach Anspruch 1 mit einem innerhalb des Luftspalts des Magnetkreises (1; 21; 31; 41; 51; 91) gebildeten Spaltraum (2; 42; 52; 92), der über mindestens eine Gasleitung (5, 6; 45, 49; 55, 56; 78, 79; 95, 96) mit einem Wechselströmungsgeber (7; 47; 57; 77; 87; 97; 107) zum abwechselnden Einbringen des Mess- und des Vergleichsgases verbunden ist und mindestens eine Ableitung (9; 59b) aufweist, sowie mit mindestens einer Wicklung (3; 4; 24; 34) für die Erfassung der periodischen Änderungen des magnetischen Flusses im Magnetkreis, dadurch gekennzeichnet, dass

a) der Spaltraum (2; 42; 52; 92) auf zwei gegenüberliegenden Seiten mit je einer Gasleitung (5,6; 45, 49; 55, 56; 78, 79; 95, 96) versehen ist, von denen die eine die Messgaszuleitung und die andere die Vergleichsgaszuleitung ist,

b) durch den Wechselströmungsgeber (7; 47; 57; 77; 87; 97; 107) ein solches Gasvolumen bewegbar ist, dass der Spaltraum (2; 42; 52; 92) periodisch abwechselnd im wesentlichen mit dem Messgas oder mit dem Vergleichsgas füllbar ist, und

c) die mindestens eine Ableitung (9; 59b) über den Spaltraum (2; 42; 52; 92) ständig mit mindestens einer der beiden Gasleitungen (5,6; 45, 49; 55, 56; 78, 79; 95, 96) in Verbindung steht.

3. Vorrichtung nach Anspruch 2, dadurch gekennzeichnet, dass der Wechselströmungsgeber (7; 57; 77) zwischen den Gasleitungen (5, 6; 55, 56) angeordnet ist und zwei durch eine Membran (8; 58; 72) getrennte Kammern aufweist, von denen die eine mit der Gasleitung (5; 55) für das Messgas und die andere mit der Gasleitung (6; 56) für das Vergleichsgas verbunden ist, derart, dass durch eine periodisch oszillierende Membranbewegung Mess- und Vergleichsgas im Wechselrhythmus in den Spaltraum einführbar und wieder aus diesem entfernbar sind.

4. Vorrichtung nach Anspruch 3, dadurch gekennzeichnet, dass sowohl in den Zuleitungen zu den Gasleitungen (5, 6; 45, 55, 56; 95, 96) als auch in der mindestens einen Ableitung (9; 59b) je eine Strömungsdrossel ($D_M$, $D_V$, $D_M'$, $D_V'$) angeordnet ist.

5. Vorrichtung nach Anspruch 2, dadurch gekennzeichnet, dass der Wechselströmungsgeber (7; 47; 57; 77; 87; 97) ventillos ausgebildet ist.

6. Vorrichtung nach Anspruch 2, dadurch gekennzeichnet, dass die der Öffnung zum Spaltraum abgewandte zweite Öffnung einer der Gasleitungen (49) des Spaltraums (41) vom Messgas bzw. Vergleichsgas umspült ist.

## Revendications

1. Procédé pour mesurer la différence de concentration de constituants paramagnétiques entre un gaz à mesurer et un gaz de comparaison par introduction périodiquement alternée des deux gaz dans l'entrefer d'un circuit magnétique et par mesure des variations périodiques du flux magnétique régnant dans le circuit magnétique, qui sont provoquées par les gaz, caractérisé en ce que:

a) on introduit les deux gaz dans l'entrefer, en alternance périodique, dans des sens opposés;

b) on déplace périodiquement une zone limite entre les deux gaz en va-et-vient dans l'entrefer, perpendiculairement aux lignes de force du champ magnétique; et

c) on laisse continuellement s'échapper une partie de mélange gazeux de la région de l'entrefer en continuant à introduire au moins l'un des deux gaz.

2. Dispositif pour la mise en œuvre du procédé selon la revendication 1, comprenant une capacité d'entrefer (2; 42; 52; 92) formée à l'intérieur de l'entrefer du circuit magnétique (1; 21; 31; 41;

51; 91) qui est en communication par l'intermédiaire d'au moins une conduite de gaz (5, 6; 45, 49; 55, 56; 78, 79; 95, 96) avec un générateur d'écoulement alternatif (7; 47; 57; 77; 87; 97; 107) pour introduire en alternance le gaz étudié et le gaz de comparaison, et qui présente au moins une conduite de sortie (9; 59b) ainsi qu'au moins un enroulement (3; 4; 24; 34) destiné à capter les variations périodiques du flux magnétique dans le circuit magnétique, caractérisé en ce que:

a) la capacité d'entrefer (2; 42; 52; 92) est munie respectivement sur deux côtés opposés, de deux conduites de gaz (5, 6; 45, 49; 55, 56; 78, 79; 95, 96) dont l'une est la conduite d'arrivée du gaz à mesurer et l'autre la conduite d'arrivée du gaz de comparasion;

b) le générateur d'écoulement alternatif (7; 47; 57; 77; 87; 97; 107) met en mouvement un volume gazeux tel que la capacité d'entrefer (2; 42; 52; 92) puisse être sensiblement remplie, en alternance remplie, en alternance périodique, du gaz à mesurer et du gaz de comparaison; et

c) la conduite de sortie ou les conduites de sortie (9; 59b) est ou sont continuellement en communication avec au moins l'une des deux conduites de gaz (5, 6; 45, 49; 55, 56; 78, 79; 95, 96) à travers la capacité d'entrefer (2; 42; 52; 92).

3. Dispositif selon la revendication 1, caractérisé en ce que le générateur d'écoulement alternatif (7; 57; 77) est disposé entre les conduites de gaz (5, 6; 55, 56) et présente deux chambres séparées par une membrane (8; 58; 72) dont l'une est en communication avec la conduite de gaz (5; 55) utilisée pour le gaz à mesurer et l'autre avec la conduite de gaz (6; 56) utilisée pour le gaz de comparaison, de telle sorte que, sous l'effet d'un mouvement oscillant périodique de la membrane, on peut introduire le gaz à mesurer et le gaz de comparaison dans la capacité d'entrefer au rythme de l'alternance et évacuer à nouveau chaque gaz de cette capacité au même rythme.

4. Dispositif selon la revendication 3, caractérisé en ce qu'un étranglement d'écoulement ($D_M$, $D_V$, $D_M'$, $D_V'$) est agencé aussi bien dans chacune des conduites d'arrivée menant aux conduites de gaz (5, 6; 45, 55, 56; 95, 96) que dans la conduite de sortie ou chacune des conduites de sortie (9; 59b)

5. Dispositif selon la revendication 2, caractérisé en ce que le générateur d'écoulement alternatif (7; 47; 57; 77; 87; 97) est d'une construction sans soupape.

6. Dispositif selon la revendication 1, caractérisé en ce que la deuxième ouverture de l'une des conduites de gaz (49) qui est à l'opposé de l'ouverture donnant sur la capacité d'entrefer (42) est balayée par le gaz à mesurer ou par le gaz de comparaison.

**Claims**

1. A method of measuring the difference in concentration of paramagnetic components between a test gas and a comparison gas by periodically alternating introduction of the two gases into the air gap of a magnetic circuit and by detecting the periodic changes in the magnetic flux in the magnetic circuit caused by the gases, characterised in that

a) the two gases are introduced alternately from opposing directions into the air gap,

b) a boundary zone between the two gases perpendicularly to the magnetic field lines is moved to and fro periodically in the air gap, and

c) a proportion of the gas mixture is allowed to escape continuously from the region of the air gap by the afterflow of at least one of the two gases.

2. An apparatus for carrying out the method according to claim 1 with a gap space (2; 42; 52; 92) which is formed inside the air gap of the magnetic circuit (1; 21; 31; 41; 51; 91), is connected via at least one gas line (5, 6; 45, 49; 55, 56; 78, 79; 95, 96) to an alternating flow generator (7; 47; 57; 77; 87; 97; 107) for the alternate introduction of the test gas and the comparison gas and comprises at least one discharge line (9; 59b), and with at least one winding (3; 4; 24; 34) for detecting the periodic changes in the magnetic flux in the magnetic circuit, characterised in that

a) the gap space (2; 42; 52; 92) is provided on two opposing sides with a respective gas line (5, 6; 45, 49; 55, 56; 78, 79; 95, 96), of which one is the test gas supply line and the other the comparison gas supply line,

b) a volume of gas can be moved by the alternating flow generator (7; 47; 57; 77; 87; 97; 107), this volume of gas being sufficient substantially to fill the gap space (2; 42; 52; 92) with the test gas or with the comparison gas in a periodically alternating manner, and

c) the discharge line (9; 59b), of which there is at least one , is connected via the gap space (2; 42; 52; 92) continuously to at least one of the two gas lines (5, 6; 45, 49; 55, 56; 78, 79; 95, 96).

3. An apparatus according to claim 2, characterised in that the alternating flow generator (7; 57; 77) is arranged between the gas lines (5, 6; 55, 56) and comprises two chambers which are separated by a diaphragm (8; 58; 72), one of the chambers being connected to the gas line (5; 55) for the test gas and the other to the gas line (6; 56) for the comparison gas in such a way that the test gas and comparison gas can be introduced into the gap space in an alternating rhythm by a periodically oscillating movement of the diaphragm and can be removed from it again.

4. An apparatus according to claim 3, characterised in that a respective flow throttling means ($D_M$, $D_V$, $D_M'$, $D_V'$) is arranged both in the supply lines to the gas lines (5, 6; 45, 55, 56; 95, 96) and in the discharge line (9; 59b), of which there is at least one.

5. An apparatus according to claim 2, characterised in that the alternating flow generator (7; 47; 57, 77; 87; 97) is designed without valves.

6. An apparatus according to claim 2, characterised in that the second opening, remote from the opening to the gap space, of one of the gas lines (49) of the gap space (42) is flushed by test gas and comparison gas.

FIG. 1

FIG. 2

FIG. 3

0 177 629

2/4

FIG. 4

C — 21
24
C
Rv
U
LI S
U_st

D

C — 31
34
C
33
LI S
U_st

D

FIG. 5

11

FIG.6

41

42

45

49

46

N

S

M

48

47

V D

55c

51

52

56c

D_M

N

D_V

S

M

55

56

V

57

58

55a

56a

D'_M

55b 56b

D'_V

59a

60

59b

M+V

FIG.7

FIG. 8a

FIG. 8b

FIG. 8c

FIG. 8d